# EUROPEAN PATENT SPECIFICATION

(11) **EP 2 749 312 B1**
(45) Date of publication and mention of the grant of the patent: **15.04.2020**
(21) Application number: 13199560.7
(22) Date of filing: 24.12.2013
(51) Int. Cl.: A61M 25/00, A61M 25/01

(54) **Catheter connector**
Katheterverbindungsstück
Connecteur de cathéter

(30) Priority: 31.12.2012 US 201213731149
(43) Date of publication of application: 02.07.2014
(73) Proprietor: Biosense Webster (Israel) Ltd., Yokneam, 2066717 (IL)
(72) Inventor: Govari, Assaf, 34400 Haifa (IL); Beeckler, Christopher Thomas, Brea, CA 92821 (US)
(74) Representative: Carpmaels & Ransford LLP

(56) References cited:
- EP-A1- 1 033 107
- GB-A- 1 048 358
- GB-A- 2 494 147
- US-A- 3 807 404
- US-A1- 2007 178 717
- US-A1- 2009 192 441
- US-A1- 2009 306 655
- US-A1- 2013 324 973

## Description

### FIELD OF THE INVENTION

The present invention relates generally to connectors, and specifically to connectors intended for use in a medical procedure.

### BACKGROUND OF THE INVENTION

A catheter typically requires a relatively large number of conductors within the catheter tube, for the purpose of conveying signals between distal and proximal ends of the catheter. Connecting the conductors at the proximal end of the catheter may require connectors that are demanding in their specifications, in order to meet all the requirements of medical equipment that is used in an invasive procedure. An alternative type of connector would be advantageous.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

EP1033107 (A1) describes a deflectable catheter comprising a catheter body, a tip section and a control handle. The control handle, which is preferably curved, comprises a housing having a generally hollow interior, a piston receiving assembly at least partially within the interior of the housing, and a piston. A puller wire extends from the control handle, through a lumen in the catheter body and into a lumen in the tip section.

US3807404 (A) describes a probe unit for applying high level high frequency voltages to tissues to be treated has a tubular handpiece having an operative end and a female connector fixed at the operative end and connectable to a source of high level high frequency voltage. The probe tip has an elongate insulating cylindrical shell having two axial slots. A resilient conductive wire, which forms part of the external cutting tip, extends partially interiorly of the shell to form two spaced opposing resilient arcuate portions which pass through the respective slots and which are depressible into the shell.

GB1048358 (A) describes a dental drilling machine consisting of an electric motor, cooled with compressed air, and arranged in a cylindrical housing one end face of which is provided with electrical connections for the supply of current to the motor and with an aperture for the throughflow of cooling air and is covered by a cap, and whose other end face has the shaft of the motor projecting through it, and of a handpiece adapted to be fitted on the motor shaft. The machine is characterized by having the electrical connection leads and the lead for the compressed air taken into the cap and terminating therin the electric connection leads terminating at electric contact elements in the cap, which are constructed as associated contacts with the electric connection contacts at one end face of the motor housing.

US2009192441 (A1) describes a surgical tool such as a liposuction cannula, equipped with a sensor at the tool tip.

US2009306655 (A1) describes a catheter suitable for medical procedures such as cardiac ablation. The catheter includes a front-loaded catheter tip with an electrically active element. The catheter can include an elongate catheter shaft assembly having an inner shaft member with a distal end and a proximal end, and an outer shaft member with a distal end, a proximal end, and a lumen between the distal end and the proximal end. The inner shaft member can be inserted into the lumen of the outer shaft member along a longitudinal direction. The catheter tip member includes at least one electrically active element, and the proximal end of the inner shaft member has at least one electrode disposed on the external surface of the shaft member, such that when inserted into a handle, the electrode electrically contacts an electrical connector element.

US2007178717 (A1) describes an electrical connector including a female portion including one or more electrical contacts and a male portion including one or more electrical contacts. The female portion and the male portion each have a self-orientating geometry that allows the male portion to be mated with the female portion in any rotational position along 360 degrees of rotation.

US2013324973 (A1) describes a medical device handle assembly including a modular extension that allows for the addition of any number of connector ports to an existing handle. GB2494147 (A) describes a handle is suitable for attachment to a medical device such as a laryngoscope. The handle comprises a housing arranged to be secured to a medical device and an insert member comprising a power source arranged to be received in the housing, the power source including a switch for switching the power source between an on and off configuration, wherein the insert member and housing are arranged such that when the insert member is received in the housing the power source switch aligns with an actuation zone of the housing such that actuation of the actuation zone causes corresponding actuation of the power source switch.

### SUMMARY OF THE INVENTION

An embodiment of the present invention provides a catheter handle, including:
a housing, configured to be gripped by an operator of a catheter coupled to the housing during a medical procedure using the catheter, the housing being formed of a first polymer and having an aperture formed therein;
an insert, formed of a second polymer, configured to be introduced into the aperture so as to mate therewith; and
a plurality of contacts, configured to be implanted into the insert in a preset spatial relationship.

Typically, the first polymer has a housing hardness, and the second polymer has an insert hardness that is harder than the housing hardness. In a disclosed embodiment, the housing hardness is within a range of 70 - 80 on a Rockwell M scale of hardness. In another disclosed embodiment the insert hardness is greater than or equal to 95 on a Rockwell M scale of hardness.

In an alternative embodiment the first polymer consists of a polycarbonate, and the second polymer consists of a glass-filled epoxy resin.

In another alternative embodiment the first polymer enables dimensions of the housing to be configured to a housing accuracy, and the second polymer enables dimensions of the preset spatial relationship to be configured to a relationship accuracy more exact than the housing accuracy.

There is further provided, according to an embodiment of the present invention, a connector, including:
a housing having an aperture and including a recess;
a plurality of contact pins enclosed by the housing and configured to convey signals, the contact pins having contact-proximal and contact-distal ends, the contact-proximal ends being implanted into the recess so that the contact-distal ends are at a contact-pin distance from the aperture;
a multiplicity of guard pins enclosed by the housing and configured not to convey signals, the guard pins having guard-proximal and guard-distal ends, the guard-proximal ends being implanted into the recess so that the guard-distal ends are at a guard-pin distance from the aperture, the guard-pin distance being smaller than the contact-pin distance.

In a disclosed embodiment the guard pins consist of non-conductive material.

In an alternative disclosed embodiment at least some of the guard pins consist of conductive material which is grounded.

There is further provided, according to an embodiment of the present inventions, a method for forming a handle of a catheter, including:
configuring a housing to be gripped by an operator of the catheter, that is coupled to the housing during a medical procedure using the catheter;
forming the housing of a first polymer and to have an aperture in the housing;
introducing an insert, formed of a second polymer, into the aperture so as to mate therewith; and
implanting a plurality of contacts into the insert in a preset spatial relationship.

There is further provided, according to an embodiment of the present invention, a method for forming a connector, including:
providing a housing having an aperture and comprising a recess;
enclosing a plurality of contact pins within the housing;
configuring the contact pins to convey signals, the contact pins having contact-proximal and contact-distal ends;
implanting the contact-proximal ends into the recess so that the contact-distal ends are at a contact-pin distance from the aperture;
enclosing a multiplicity of guard pins within the housing;
configuring the guard pins not to convey signals, the guard pins having guard-proximal and guard-distal ends; and
implanting the guard-proximal ends into the recess so that the guard-distal ends are at a guard-pin distance from the aperture, the guard-pin distance being smaller than the contact-pin distance.

There is further provided, according to an embodiment of the present invention, a connector, including:
a housing having an aperture and including a recess;
a plurality of contact pins enclosed by the housing and configured to form a contact with a patient undergoing a medical procedure, the contact pins having contact-proximal and contact-distal ends, the contact-proximal ends being implanted into the recess so that the contact-distal ends are at a contact-pin distance from the aperture;
a multiplicity of guard pins enclosed by the housing and configured to be isolated from the patient, the guard pins having guard-proximal and guard-distal ends, the guard-proximal ends being implanted into the recess so that the guard-distal ends are at a guard-pin distance from the aperture, the guard-pin distance being smaller than the contact-pin distance.

The guard pins may be configured to convey signals between a first circuit not in electrical contact with the patient and a second circuit not in electrical contact with the patient.

There is further provided, according to an embodiment of the present invention, a method for forming a connector, including:
providing a housing having an aperture;
forming a recess in the housing;
enclosing a plurality of contact pins within the housing;
configuring the contact pins to form a contact with a patient undergoing a medical procedure, the contact pins having contact-proximal and contact-distal ends;
implanting the contact-proximal ends into the recess so that the contact-distal ends are at a contact-pin distance from the aperture;
enclosing a multiplicity of guard pins within the housing;
isolating the guard pins from the patient, the guard pins having guard-proximal and guard-distal ends;
implanting the guard-proximal ends into the recess so that the guard-distal ends are at a guard-pin distance from the aperture, the guard-pin distance being smaller than the contact-pin distance.

The present disclosure will be more fully understood from the following detailed description of the embodiments thereof, taken together with the drawings, in which:

### BRIEF DESCRIPTION OF THE DRAWINGS

Fig. 1 is a schematic illustration of catheter insertion apparatus, according to an embodiment of the present invention;
Fig. 2 is a schematic exploded diagram of two parts of the apparatus of Fig. 1 connected together, according to an embodiment of the present invention;
Fig. 3 is a schematic diagram of a first portion of a handle, according to an embodiment of the present invention;
Fig. 4 is a schematic diagram of views of an insert, according to an embodiment of the present invention;
Fig. 5 is a schematic exploded diagram of a connector connected to a cable, according to an embodiment of the present invention; and
Fig. 6 is a schematic exploded illustration of a portion of a connector, according to an alternative embodiment of the present invention.

### DETAILED DESCRIPTION OF EMBODIMENTS

### OVERVIEW

An embodiment of the present invention provides a catheter connector that is produced in two parts: a housing, and an insert configured to be introduced into the housing. Contacts for the connector are implanted into the insert in a preset spatial relationship, so that, for example, the contacts may be configured to be symmetrically positioned within a hexagon.

The housing is formed of a first polymer, typically a polycarbonate, and typically by injection molding. The housing is formed with an aperture into which the insert is positioned, and the insert and aperture are designed to mate with each other.

The insert is formed of a second polymer, typically a glass-filled epoxy resin, into which the contacts of the connector are fixedly positioned.

The two polymers are typically selected to have different hardness values, the polymer of the insert being harder than the polymer of the housing. By selecting the insert hardness to be relatively high, dimensions of the spatial relationship of the contacts may be maintained, even under repeated mating cycles of the connector. Having the housing to be softer than the insert does not affect the dimensional stability of the contacts within the insert, but may lead to a significant reduction in cost of formation of the complete connector, since the softer polymer is typically less costly than the harder polymer.

In an alternative embodiment of the present invention, a connector is formed of a one-piece housing with an aperture, the housing also having a recessed portion, herein also termed a recess. Contact pins for the connector are embedded into the recess, and are configured to convey signals.

Typically, in addition to the contact pins, guard pins, configured not to carry signals, are also embedded into the recess. The ends of the guard pins are configured to be closer to the aperture than the ends of the contact pins.

Alternatively, the guard pins may be configured to carry, or convey, signals, but the guard pins are isolated from the contact pins, so that the signals conveyed by the guard pins cannot be conveyed by the contact pins.

Use of guard pins, as described herein, may be useful during a medical procedure using the connector. For example, in a cardiac procedure the contact pins may be directly connected electrically with a patient undergoing the procedure. During such a procedure, the presence of guard pins may prevent inadvertent contact via the aperture of a finger of a person using the connector (or of any other object) from touching the contact pins, so possibly having a critical effect on the patient.

### SYSTEM DESCRIPTION

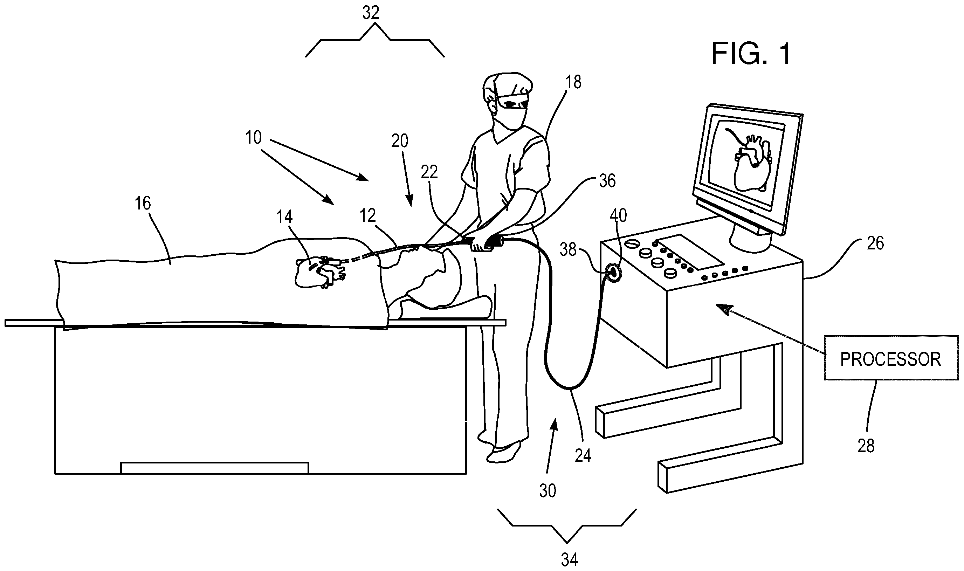

Reference is now made to Fig. 1, which is a schematic illustration of catheter insertion apparatus 10, according to an embodiment of the present invention. Apparatus 10 may be used to insert a catheter 12 into a body cavity 14 of a patient 16 during a medical procedure performed on the patient. The procedure is performed by an operator 18, typically a medical professional, of apparatus 10. Catheter 12 is typically a tube comprising, *inter alia,* a plurality of wires 20 that traverse the tube. Wires 20 are typically configured to transfer signals between a distal end and a proximal end of the catheter. U.S. Patent 7,848,789, to Govari et al., whose disclosure is incorporated herein by reference, describes insertion of a catheter such as catheter 12 into the body cavity of a patient.

The type of signals transferred depends on the procedure being performed. For example, if the procedure comprises an electrophysiological investigation of the heart, the signals typically include electrical signals generated by the heart, as well as signals generated by tracking devices positioned within the distal tip of the catheter. Depending on the type of catheter used, the signals may also include signals representative of a temperature of the distal tip of the catheter, and signals representative of a force on the distal tip.

In order to insert catheter 12, the catheter is coupled at its proximal end to a handle 22, which is gripped by operator 18 during the procedure. Using the handle, the operator is able to insert catheter 12 into patient 16, to manipulate the catheter within the patient, and to withdraw the catheter from the patient.

A cable 24 is connected between handle 22 and a console 26, the cable serving to transfer the signals referred to above between the handle and the console. Console 26 comprises a processor 28, which together with other digital and/or analog devices operates to provide signals for transmission to the distal end of the catheter, as well as to receive signals from the distal end. The signals are transferred within cable 24 on a plurality of wires 30, the number of wires 30 being equal to the number of wires 20 (in catheter 12).

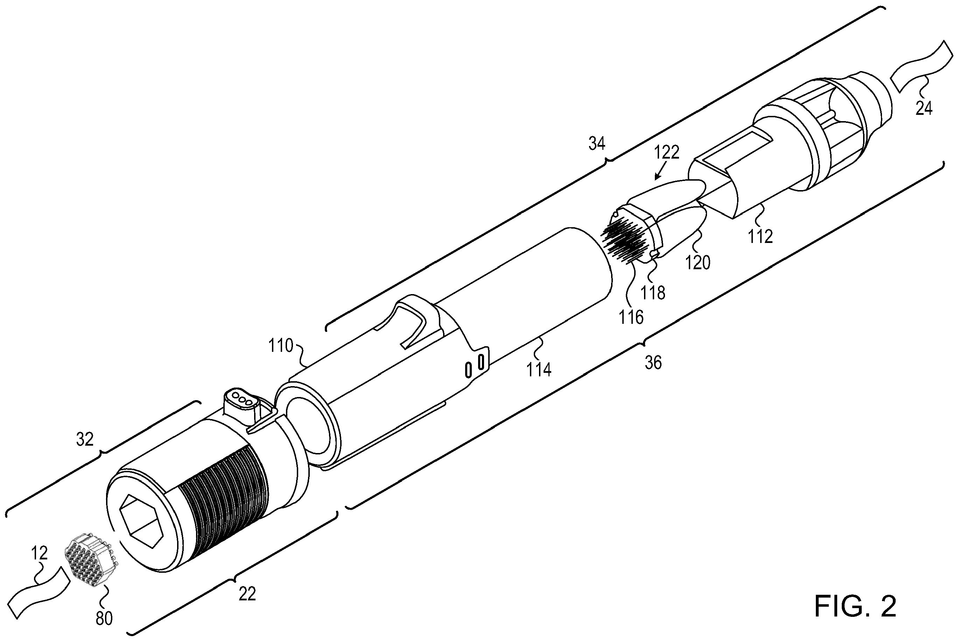

For ease of assembly and disassembly of apparatus 10, catheter 12 and handle 22 are typically constructed to be a first single unit 32. Cable 24 is constructed as a second single unit 34, having a first connector 36 mating with the handle, and a second connector 38 mating with a socket 40 on console 26. Apparatus 10 comprises the first and second single units. Handle 22 and its connection to cable 24 are shown in more detail in Fig. 2 below.

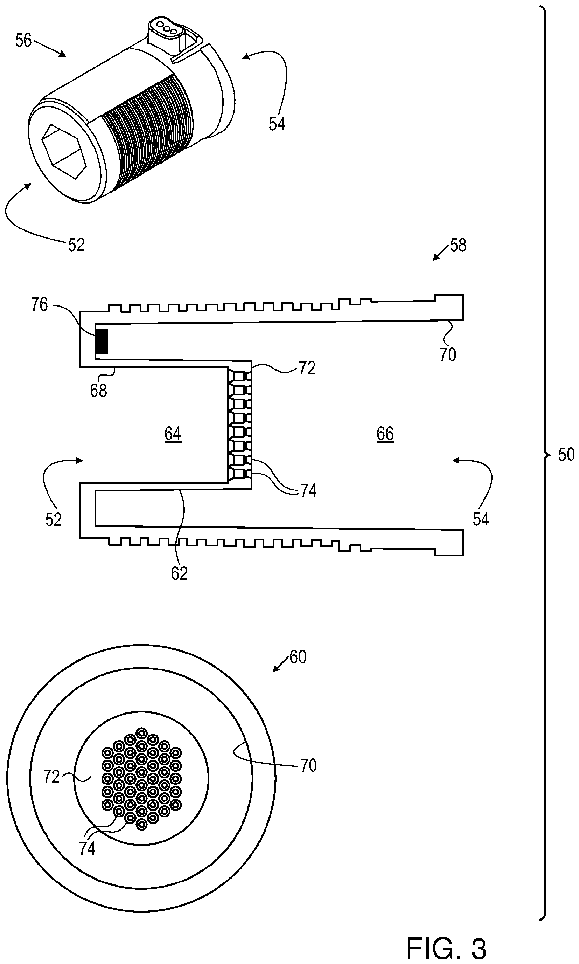

Fig. 2 is a schematic exploded diagram of part of unit 32 connected to part of unit 34, and Fig. 3 is a schematic diagram of a first portion of handle 22, according to embodiments of the present invention. Handle 22 is comprised of an approximately cylindrical handle housing 50 having an approximately hexagonal distal aperture 52 and an approximately circular proximal aperture 54. Three views of housing 50 are shown in Fig. 3: a perspective view 56, a cross-sectional view 58, and a proximal view 60, taken through proximal aperture 54. Housing 50 is constructed, typically by injection molding, of a relatively soft polymeric material. In one embodiment of the present invention, housing 50 is formed of a polycarbonate such as Makrolon 2458, having a Rockwell M scale hardness of approximately 75. However, housing 50 may be formed of any other convenient polymer having a Rockwell M scale hardness that lies in a range that is approximately 70 - 80, so that the housing is relatively soft.

Housing 50 is divided internally by a divider 62, which separates the housing into two internal sections, a distal internal volume 64 opening to distal aperture 52, and a proximal internal volume 66 opening to proximal aperture 54. Walls 68 of volume 64 are approximately hexagonal in shape, corresponding to aperture 52. Walls 70 of volume 66 are approximately cylindrical in shape. A base 72 of divider 62 is approximately circular in shape, and there is a plurality of through holes 74 penetrating the base, the holes being arranged in a preset spatial relationship. In one embodiment, the preset spatial relationship comprises 44 holes arranged symmetrically within a hexagon. The following description, except where otherwise indicated, assumes by way of example that the preset spatial relationship is of 44 holes arranged symmetrically within a hexagon. In some embodiments housing 50 comprises a circuit 76, an exemplary function of which is described below.

Handle 22 also has a second portion, comprising an approximately hexagonal insert 80 which is configured to mate with aperture 52.

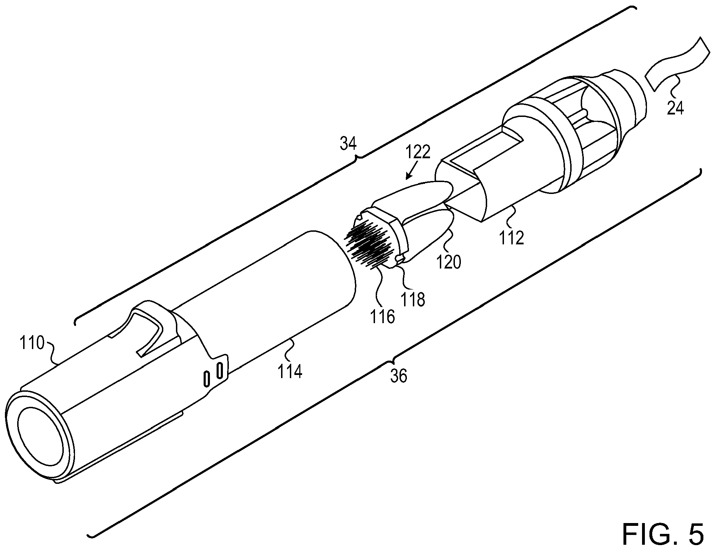

Other elements of apparatus 10 that are identified in Fig. 2 are described with reference to Fig. 5 below.

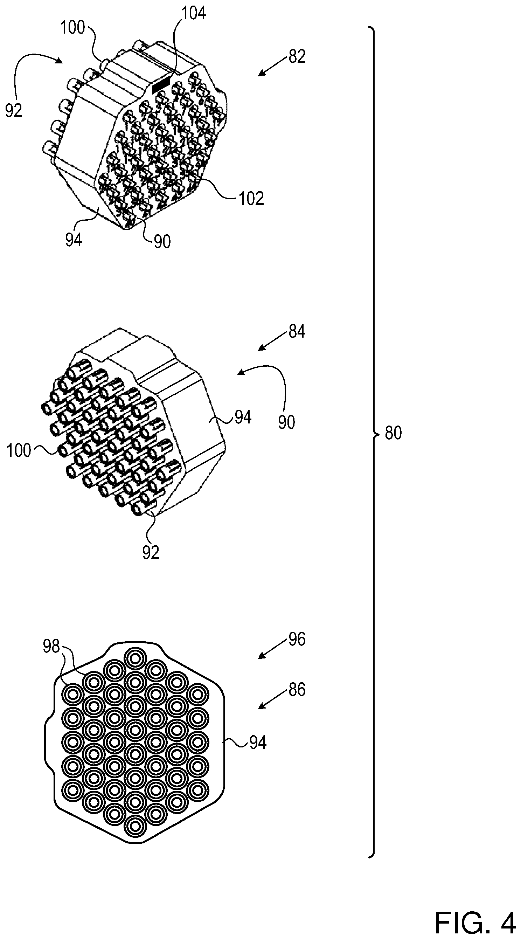

Fig. 4 is a schematic diagram of views of insert 80, according to an embodiment of the present invention. Fig 4 shows first and second perspective views 82, 84, and a cross-section view 86, of insert 80. Insert 80 has two faces, a distal face 90 and a proximal face 92, and is configured so that the proximal face of the insert faces towards cable 24 (Fig. 2).

Insert 80 comprises a substrate 94, which is constructed, typically by molding or machining a panel of identical parts, of a polymeric material which is harder than the material of housing 50. In one embodiment of the present invention, substrate 94 of the insert is formed of a glass-filled epoxy resin such as FR4 glass-filled epoxy, which has a Rockwell M scale hardness of approximately 95 or more (as compared to the hardness of 75 for the embodiment of housing 50 described above). However, substrate 94 may be formed of any other convenient polymer that is harder than the polymer of housing 50.

Substrate 94 has a plurality of through holes 96 arranged in the preset spatial relationship described above, i.e., there are 44 holes 96, and within holes 96 substantially similar electrically conductive contacts 98 are fixedly implanted into the substrate. In the embodiment described herein, each contact 98 is assumed to comprise a female socket 100 with a solder cup 102, and the contacts are implanted into holes 96 so that the female sockets protrude from proximal face 92, and so that the solder cups protrude from distal face 90.

To assemble unit 32, wires 20 of cable 12 are soldered to solder cups 102 of insert 80. The insert is then fixedly positioned within distal volume 64 of housing 50, and is located within volume 64 so that female sockets 100 mate with and enter holes 74 of base 72. Sockets 100 are configured to enter holes 74, but to remain recessed in the holes, so as not to penetrate into internal volume 66. Thus, inadvertent entry of a conducting object, such as a finger of operator 18, into volume 66, will not introduce signals to sockets 100, even if the finger touches the wall of base 72 (on the internal volume 66 side) of the handle divider, since the base provides insulation between the finger and the sockets. Positioning insert 80 within volume 64 forms handle 22. In some embodiments insert 80 comprises a circuit 104, an exemplary function of which is described below.

Fig. 5 is a schematic exploded diagram of connector 36 connected to cable 24, according to an embodiment of the present invention. Connector 36 comprises a first connector housing 110, which mates with handle 22. First connector housing 110 connects to a second connector housing 112 so as to enclose other elements of connector 36. The other elements enclosed by the two housings comprise a mu-metal shield 114, a set of male pins 116 implanted in a substrate 118, and a flexible printed circuit board 120. Pins 116 are formed within substrate 118 in the preset spatial relationship referred to above, so that there are 44 male pins 116 fixedly implanted in substrate 118. The 44 pins 116 are soldered to 44 separate traces 122 in printed circuit board 120, and wires 30 of cable 24 are soldered to the 44 traces.

Referring back to Figs. 2 and 1, apparatus 10 is assembled by inserting connector 36 into handle 32. The insertion mates male pins 116 of connector 36 with female sockets 100 in handle 22. The mating of the pins and sockets is facilitated by holes 74 of divider 62, the holes acting to guide the pins into correct alignment with the sockets.

In order for the mating of pins 116 with sockets 100 to be successful, typically over multiple mating cycles of apparatus 10, dimensions of the spatial relationship of contacts 98, implanted in substrate 94 of insert 80, typically need to have an accuracy corresponding to the accuracy of male pins 116 in substrate 118. Embodiments of the present invention achieve and maintain the required accuracy for sockets 100 by forming substrate 94 from relatively hard polymeric material, such as is exemplified above. However, rather than forming all of handle 22 from such relatively hard material, housing 50 is formed of softer polymeric material than substrate 94. The softer polymer of housing 50 is configurable to an accuracy that is less exact, as well as being insufficiently exact, compared to the accuracy achieved by the harder polymer of substrate 94. The more exact accuracy is necessary to maintain the dimensions of the spatial relationship of contacts 98, and thus of sockets 100. However, production of items using only the softer polymer is significantly less expensive than production using only the harder polymer. The design of handle 22 as two components, an insert 80 formed of the hard polymer, and a housing 50 formed of the softer polymer, leads to substantial savings in the volume of hard polymer required, and thus to substantial financial savings in production of handle 22. It will be understood that the savings are achieved with no degradation in the accuracy provided for contacts 98 in the handle.

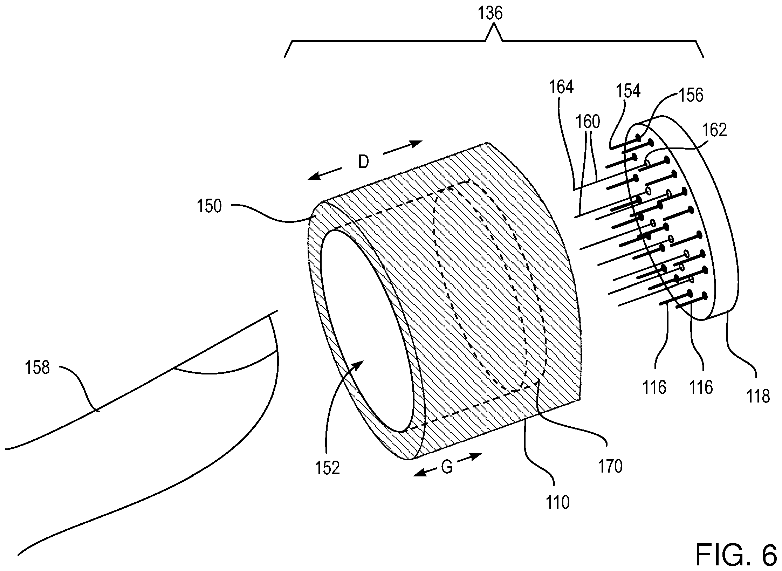

Fig. 6 is a schematic exploded illustration of a portion of a connector 136, according to an alternative embodiment of the present invention. Apart from the differences described below, the operation of connector 136 is generally similar to that of connecter 36 (Figs. 2 and 5), and elements indicated by the same reference numerals in both connectors 36 and 136 are generally similar in construction and in operation. For simplicity, only substrate 118, male pins 116, and a portion of first connector housing 110 are schematically illustrated in Fig. 6, and it will be understood that other elements of connector 136, such as those described above with reference to connector 36, are present in connector 136.

A distal end 150 of first connector housing 110 has an aperture 152. Male pins 116, also referred to herein as contact pins, are assumed to have distal ends 154 and proximal ends 156, the proximal ends being implanted in substrate 118. On assembly of connector 136, contact pins 116 remain within housing 110, so that distal ends 154 are recessed from aperture 152 by a preset distance D. However, a finger 158, such as a finger of operator 18 of apparatus 10, may inadvertently enter the aperture and contact distal ends 154, leading to possible injury to the operator and/or damage to elements in console 26.

To prevent the effects of such inadvertent contact, a multiplicity of generally similar guard pins 160 are implanted into substrate 118, guard pins 160 having proximal ends 162 and distal ends 164. The distal ends are implanted into the substrate, and the guard pins are configured so that their distal ends, while remaining within housing 110, are closer to aperture 152 than distal ends 154 of contact pins 116. Thus distal ends 164 are recessed from aperture 152 by a preset distance G, which is less than distance D, so that guard pins 160 prevent contact of finger 158 with contact pins 116.

Typically, guard pins 160 are configured to convey no signals, in contrast to contact pins 116 which do convey signals, so that the guard pins may be non-conductive. Alternatively or additionally, at least some of the guard pins may be conductive, in which case they are typically grounded.

Further alternatively or additionally, the guard pins may convey signals, but are configured to be isolated from the contact pins. For example, connector 136 may be used during a medical procedure wherein body cavity 14 is the heart of patient 16, in which case contact pins 116 may be in direct electrical contact, via sockets 100, with the heart. Referring back to Fig. 3, circuit 76 may, for example, comprise a keypad or a driver thereof installed in handle 22. Providing guard pins 160 are isolated from contact pins 116, and thus are isolated from the patient, the guard pins may be used to convey signals between circuit 76 and another circuit, such as processor 28.

In no cases do guard pins 160 convey signals to or from patient 16.

It will be appreciated that in embodiments of the present invention using guard pins such as those exemplified in the description above, corresponding holes for the guard pins, i.e., holes having the same spatial relationship as the guard pins, are made in elements mating with connector housing 110. Thus corresponding holes are formed in base 72 and typically also in substrate 94.

The description above has assumed that contact pins 116 and guard pins 160 are implanted in substrate 118, and that the substrate is separate from housing 110. In an alternative embodiment, the contact pins and the guard pins are implanted in a recessed portion 170, also referred to herein as recess 170, of housing 110. Recess 170 is formed within the housing, in which case the recess and the housing may be produced as a one-piece element. In the alternative embodiment the geometric spacing of the distal ends of the contact pins and of the guard pins, relative to aperture 152, is as described above for the embodiment using substrate 118.

The explanation above has described guard pins for connector 136. Those having ordinary skill in the art will be able to adapt the description for guard pins for sockets 100 of handle 22. In this case the guard pins are implanted into substrate 94, and penetrate through base 72 into internal volume 66. Holes corresponding to the guard pins are also made in substrate 118. It will be understood that the application of guard pins in substrate 94 may be in addition to, or in place of, the insulation provided by the recessing of sockets 100 in holes 74 of base 72. As for connector 136, guard pins for sockets 100 are isolated from the sockets, so that a patient who may be in direct electrical contact with the sockets is isolated from the guard pins. Furthermore, because the guard pins implanted into substrate 94 are isolated from sockets 100, the guard pins may be non-conductive or conductive. If the guard pins are conductive, they may be used to convey signals to or from circuit 104. Circuit 104 may, for example, comprise a non-volatile memory, such as an EEPROM (Electrically Erasable Programmable Read Only Memory), that is used to store information related to catheter 12 and that is connected to substrate 94.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A catheter handle (22), comprising:
a housing (50), configured to be gripped by an operator of a catheter coupled to the housing during a medical procedure using the catheter, the housing being formed of a first polymer and having an aperture formed therein;
an insert (80), formed of a second polymer, configured to be introduced into the aperture so as to mate therewith; and
a plurality of electrically conductive contacts (98), configured to be implanted into the insert in a preset spatial relationship,
wherein the first polymer has a housing hardness, and wherein the second polymer has an insert hardness that is harder than the housing hardness.

2. A method for forming a handle of a catheter (22) according to claim 1, comprising:
configuring a housing (50)to be gripped by an operator of the catheter, that is coupled to the housing during a medical procedure using the catheter;
forming the housing of a first polymer and to have an aperture in the housing;
introducing an insert (80), formed of a second polymer, into the aperture so as to mate therewith; and
implanting a plurality of electrically conductive contacts (98)into the insert in a preset spatial relationship.

3. The catheter handle (22) of claim 1 or the method according to claim 2, wherein the housing hardness is within a range of 70 - 80 on a Rockwell M scale of hardness.

4. The catheter handle (22) of claim 1 or the method according to claim 2, wherein the insert hardness is greater than or equal to 95 on a Rockwell M scale of hardness.

5. The catheter handle (22) according to claim 1 or the method according to claim 2, wherein the first polymer comprises a polycarbonate, and wherein the second polymer comprises a glass-filled epoxy resin.

6. The catheter handle (22) according to claim 1 or the method according to claim 2, wherein the first polymer enables dimensions of the housing (50) to be configured to a housing accuracy, and wherein the second polymer enables dimensions of the preset spatial relationship to be configured to a relationship accuracy more exact than the housing accuracy.

## Patentansprüche

1. Kathetergriff (22), umfassend:
ein Gehäuse (50), gestaltet zum Greifen durch einen Bediener eines an das Gehäuse gekoppelten Katheters während eines medizinischen Verfahrens unter Verwendung des Katheters, wobei das Gehäuse aus einem ersten Polymer gebildet ist und eine darin gebildete Öffnung aufweist;
einen Einsatz (80), der aus einem zweiten Polymer gebildet ist, gestaltet zum Einführen in die Öffnung, um damit zusammenzupassen; und
eine Vielzahl von elektrisch leitfähigen Kontakten (98), gestaltet zum Einsetzen in den Einsatz in einer vorbestimmten räumlichen Beziehung,
wobei das erste Polymer eine Gehäusehärte aufweist und wobei das zweite Polymer eine Einsatzhärte aufweist, die härter als die Gehäusehärte ist.

2. Verfahren zur Herstellung eines Griffs eines Katheters (22) gemäß Anspruch 1, umfassend:
Konfigurieren eines Gehäuses (50) zum Greifen durch einen Bediener des Katheters, der an das Gehäuse gekoppelt ist, während eines medizinischen Verfahrens unter Verwendung des Katheters;
Bilden des Gehäuses aus einem ersten Polymer, mit einer Öffnung in dem Gehäuse;
Einführen eines Einsatzes (80), der aus einem zweiten Polymer gebildet ist, in die Öffnung, um damit zusammenzupassen; und
Einsetzen einer Vielzahl von elektrisch leitfähigen Kontakten (98) in den Einsatz in einer vorbestimmten räumlichen Beziehung.

3. Kathetergriff (22) gemäß Anspruch 1 oder Verfahren gemäß Anspruch 2, wobei die Gehäusehärte in einem Bereich von 70-80 auf der Rockwell-M-Härteskala liegt.

4. Kathetergriff (22) gemäß Anspruch 1 oder Verfahren gemäß Anspruch 2, wobei die Einsatzhärte größer als oder gleich 95 auf der Rockwell-M-Härteskala ist.

5. Kathetergriff (22) gemäß Anspruch 1 oder Verfahren gemäß Anspruch 2, wobei das erste Polymer ein Polycarbonat umfasst und wobei das zweite Polymer ein glasgefülltes Epoxyharz umfasst.

6. Kathetergriff (22) gemäß Anspruch 1 oder Verfahren gemäß Anspruch 2, wobei das erste Polymer erlaubt, dass die Abmessungen des Gehäuses (50) auf eine Gehäusegenauigkeit gestaltet werden, und wobei das zweite Polymer erlaubt, dass die Abmessungen der vorbestimmten räumlichen Beziehung auf eine Beziehungsgenauigkeit gestaltet werden, die genauer als die Gehäusegenauigkeit ist.

## Revendications

1. Poignée de cathéter (22) comprenant :
un boîtier (50) configuré pour être saisi par un opérateur d'un cathéter couplé au boîtier pendant une intervention médicale utilisant le cathéter, le boîtier étant formé d'un premier polymère et ayant un orifice formé à l'intérieur de celui-ci ;
une pièce rapportée (80) formée d'un second polymère, configurée pour être introduite dans l'orifice de sorte à s'accoupler à celui-ci ; et
une pluralité de contacts électroconducteurs (98), configurés pour être implantés dans la pièce rapportée dans une relation spatiale prédéfinie,
dans laquelle le premier polymère présente une dureté de boîtier et dans laquelle le second polymère présente une dureté de pièce rapportée qui est plus dure que la dureté de boîtier.

2. Procédé pour former une poignée d'un cathéter (22) selon la revendication 1, consistant :
à configurer un boîtier (50) pour être saisi par un opérateur du cathéter, qui est couplé au boîtier pendant une intervention médicale utilisant le cathéter,
à former le boîtier d'un premier polymère et de sorte à avoir un orifice dans le boîtier ;
à introduire une pièce rapportée (80), formée d'un second polymère, dans l'orifice de sorte à s'accoupler à celui-ci ; et
à implanter une pluralité de contacts électroconducteurs (98) dans la pièce rapportée dans une relation spatiale prédéfinie.

3. Poignée de cathéter (22) selon la revendication 1 ou procédé selon la revendication 2, la dureté de boîtier se situant à l'intérieur d'une plage allant de 70 à 80 sur une échelle de dureté Rockwell M.

4. Poignée de cathéter (22) selon la revendication 1 ou procédé selon la revendication 2, la dureté de pièce rapportée étant supérieure ou égale à 95 sur une échelle de dureté Rockwell M.

5. Poignée de cathéter (22) selon la revendication 1 ou procédé selon la revendication 2, le premier polymère comprenant un polycarbonate et le second polymère comprenant une résine époxy remplie de verre.

6. Poignée de cathéter (22) selon la revendication 1 ou procédé selon la revendication 2, le premier polymère permettant que des dimensions du boîtier (50) soient configurées pour une précision de boîtier et le second polymère permettant que des dimensions de la relation spatiale prédéfinie soient configurées pour une précision de relation plus exacte que la précision de boîtier.
